# EUROPEAN PATENT SPECIFICATION

(11) **EP 1 194 137 B1**
(45) Date of publication and mention of the grant of the patent: **29.12.2004**
(21) Application number: 00937123.8
(22) Date of filing: 22.06.2000
(51) Int. Cl.: A61K 31/122, A61P 31/06

(54) **NAPHTHOQUINONE DERIVATIVES AND THEIR USE IN THE TREATMENT AND CONTROL OF TUBERCULOSIS**
NAPHTHOCHINONDERIVATE UND IHRE VERWENDUNG ZUR BEHANDLUNG UND KONTROLLE VON TUBERKULOSE
DERIVES DE NAPHTOQUINONE ET LEURS UTILISATIONS DANS LE TRAITEMENT ET L'ERADICATION DE LA TUBERCULOSE

(30) Priority: 24.06.1999 ZA 9904176
(43) Date of publication of application: 10.04.2002
(73) Proprietor: University of Pretoria, Hatfield, 0083 Pretoria (ZA); South African Medical Research Council, 7505 Parow (ZA)
(72) Inventor: MEYER, Jacobus Johannes Marion, 0047 Pretoria (ZA); LALL, Namrita, 0083 Pretoria (ZA)
(74) Representative: Samuels, Lucy Alice
(86) International application number: PCT/IB2000/000837
(87) International publication number: WO 2001/000554

(56) References cited:
- ADENIYI, B. A. ET AL: "Antibacterial activity of diospyrin, isodiospyrin and bisisodiospyrin from the root of Diospyros piscatoria (Gurke) (Ebenaceae)" PHYTOTHER. RES. (2000), 14(2), 112-117 , XP000978371
- KHAN M R ET AL: "ANTIBIOTIC ACTION OF CONSTITUENTS OF ROOT BARK OF EUCLEA-NATALENSIS." PAK J SCI IND RES, (1978 (RECD 1979)) 21 (5-6), 197-199. , XP000978450
- KHAN, M. R. (1) ET AL: "Constituents of Diospyros lolin, D. maritima and D. novoguinensis." FITOTERAPIA, (APRIL, 1999) VOL. 70, NO. 2, PP. 194-196. , XP000978591
- DATABASE CHEMABS [Online] CHEMICAL ABSTRACTS SERVICE, COLUMBUS, OHIO, US; VICHKANOVA, S. A. ET AL: "Search for antimicrobial drugs among quinones of plant origin" retrieved from STN Database accession no. 91:83030 XP002157353 & RASTIT. RESUR. (1979), 15(2), 167-77 ,
- ROUSHDI I M ET AL: "Synthesis of 1.4-naphthoquinones-4-aryl(aroyl)hydrazone s of potential antimicrobial activity." PHARMAZIE, (1976) 31 (12) 856-9. , XP000971908
- HAZRA, BANASRI ET AL: "In vitro antiplasmodial effects of diospyrin, a plant-derived naphthoquinoid, and a novel series of derivatives" PHYTOTHER. RES. (1995), 9(1), 72-4 , XP000978372
- YARDLEY, VANESSA ET AL: "In vitro activity of diospyrin and derivatives against Leishmania donovani, Trypanosoma cruzi and Trypanosoma brucei brucei" PHYTOTHER. RES. (1996), 10(7), 559-562 , XP000978369
- HAZRA, BANASRI ET AL: "Biological activity of diospyrin towards Ehrlich ascites carcinoma in Swiss A mice" PLANTA MED. (1984), 50(4), 295-7 , XP000978377
- HAZRA, BANASRI ET AL: "New diospyrin derivatives with improved tumor inhibitory activity towards Ehrlich ascites carcinoma" MED. SCI. RES. (1994), 22(5), 351-3 , XP000978374
- OERIU I: "Relation between the chemical structure and the antitubercular effect of alpha-naphthoquinone derivatives substituted in 2 and 3 positions." PHARMAZIE, (1961 MAY) 16 266-72., XP000971910
- OERIU I: "Zusammenhänge zwischen der chemischen Struktur und der antituberkulösen Wirkung der in Stellung 2 und 3 substituieren Derivate des alpha-Naphthochinons" PHARMAZIE,DD,VEB VERLAG VOLK UND GESUNDHEIT. BERLIN, no. 16, 1961, pages 320-327, XP002078405 ISSN: 0031-7144
- TIKKANEN L. ET AL: 'UTAGENICITY OF NATURAL NAPHTHOQUINONES AND BENZOQUINONES IN THE SALMONELLA/MICROSOME TEST' MUTATION RESEARCH vol. 124, 1983, pages 25 - 34
- MAHONEY N. ET AL: 'EGULATION OF AFLATOXIN PRODUCTION OF NAPHTHOQUINONES OF WALNUT' J. AGRIC. FOOD CHEM. vol. 48, 2000, pages 4418 - 4421
- LIKHITWITAYAWUID K. ET AL: 'NTIMALARIAL NAPHTHOQUINONES FROM NEPENTS THOARELII2' PLANTA MEDICA vol. 64, 1998, pages 237 - 241

## Description

### BACKGROUND OF THE INVENTION

THIS invention relates to the use of naphthoquinone derivatives of formula 1a or 1b as defined in claim 1 in the manufacture of a medicament for the treatment and control of tuberculosis caused by *Mycobacterium tuberculosis*.

Tuberculosis (TB) remains a serious health problem in many regions of the world, especially in developing nations. It is a contagious disease and is becoming epidemic in some parts of the world. It is estimated that 30-60% of adults in developing countries are infected with *Mycobacterium tuberculosis.* Approximately 8-10 million individuals develop clinical TB and 3 million die of TB each year (WHO/IUATLD, 1989).

In South Africa, over 3 in every thousand people die of TB, the highest rate in the world, while one out of every 200 people suffers from active tuberculosis. Tuberculosis is the most commonly notified disease in South Africa and the fifth largest cause of death among the black population (South African Tuberculosis Association, 1998).

In the United States, the number of TB cases steadily decreased until 1986 when an increase was noted. Since then TB cases have continued to rise. Ten million individuals are infected in the U.S.A., with approximately 26000 new cases of active disease each year (National Jewish Medical and Research Center, 1994).

Individuals infected with Human Immunodeficiency Virus (HIV) are very susceptible to tuberculosis and often develop this disease before other manifestations of AIDS become apparent (Grange and Davey, 1990). Control of the TB epidemic linked with HIV infection will depend largely on the adequate treatment of TB, and possibly of effective chemoprophylaxis, not just for HIV-infected persons but for communities as well (WHO/IUATLD, 1989).

TB therapy has been revolutionized and the present treatment regimes for TB are based on multidrug therapy with usually 3 or 4 antituberculosis drugs. However, the problem of multidrug resistant tubercle bacilli is emerging for various drugs such as isoniazid, ethambutol, rifampicin and streptomycin, for example (Girling, 1989; Grange and Davey, 1990). Drug-resistant TB is very difficult to treat requiring greater numbers and varieties of medications for a longer period of treatment. The need for new antituberculosis agents is urgent due to the increasing resistance of mycobacteria to these classic antituberculosis drugs. A recent WHO report states that, globally, 2% of all cases of tuberculosis are multidrug resistant - by definition, resistance to rifampicin plus isoniazid (plus/minus other resistances). Such cases can be treated in the USA and other high resource regions but at a great cost (> US$ 250,000 per case!) and using very long courses of rather toxic drugs, thereby raising serious problems of compliance (WHO, 1997). South Africa is witnessing an explosion in the number of cases of drug-resistant tuberculosis. In some parts of South Africa, 1 in 10 cases of TB is resistant to treatment (New Scientist, March 1997). It is essential to have new antituberculosis agents, preferably those that can readily and simply be produced from some local source.

Various petroleum ether and then chloroform extracts of *Euclea natalensis* root bark have previously been investigated and shown to have marked antibiotic activity against *Staphylococcus aureus*. 7-Methyljuglone, mamegakinone and diospyrin were isolated and found active against a number of pathogenic organisms including *Neisseria gonarrhoeae* and *Shigella Spp*., although a triterpenoid lupeol isolated from *E*. *natalensis* was found to be inactive against *Staphylococcus aureus.* (KHAN, M. R. et al: 'Antibiotic action of constituents of root bark of *Euclea-Natalensis*', Pak J Sci Ind Res, (1978 (reed 1979)) 21 (5-6), 197-199., XP000978450).

An investigation into the antibacterial activity of *D*.*maritima* and *D*.*novoguinensis* confirmed it to be due to the presence of 7-methyljuglone and plumbagin. (KHAN, M. R. et at: 'Constituents of Diospyros *lolin*. *D*.*maritima* and *D*.*novoguinensis*', Fitoterapia, (April, 1999), Vol. 70, No. 2. pp. 194-196., XP000978591).

A study of the antimicrobial activity of 191 preparations from 70 plant species and of the antiviral activity of 22 quinones, showed that plumbagin from *Phumbago europea* and *Ceratostigma plumbaginsides* was active against gram-positive and gram-negative bacteria, *Mycobacterium tubericulosis*, *Microsporum lanesum*, *Candida albicans*, *Entamoebo histolytica*, and *Trichomonas vaginalis*. (VICHKANOVA, S.A. et al: 'Search for antimicrobial drugs among quinones of plant origin' retrieved from STN Database accession no. 91:83030 XP002157353 & Rastit. Resur. (1979), 15(2), 167-77).

The potential antiplasmodial effects of a plant-derived bis-naphthoquinoid, diospyrin (from *Diospyros Montana* Roxb.) and some semisynthetic derivatives thereof have also previously been investigated. (HAZRA, Banasri et al: 'In vitro antiplasmodial effects of diospyrin, a plant-derived naphthoquinoid, and a novel series of derivatives', Phytother. Res. (1995), 9(1), 72-4, XP000978372).

The *in vitro* activity of diospyrin and derivatives thereof against *Leishmania donovani*, *Trypanosoma cruzi* and *Trypanosoma brucei brucei,* have also shown potential in the treatment of the parasitic protozoan disease leishmaniasis and trypanosomiasis. (YARDLEY, Vanessa et al: '*In vitro* activity of diospyrin and derivatives against Leishmania *donovani*, *Trypanosoma cruzi* and *Trypanosoma brucei brucei*, Phytother. Res. (1996), 10(7), 559-562, XP000978369).

Diospyrin has been isolated from the bark of *Diospyros Montana* Roxb., purified and *in vitro* and *in vivo* experiments carried out to study its biological activity against *Ehrlich Ascites Carcinoma* in Swiss A mice. The investigations indicated that further investigation in order to explore their potential as antitumour agents was deserved. (HAZRA, Banasri et al: 'Biological activity of diospyrin towards *Ehrlich ascites carcinoma* in Swiss A mice', Plant Med. (1984), 50(4), 295-7, XP000978377).

In view of the known tumour-inhibitory activity of diospyrin against *Ehrlich ascites* carcinoma, derivatives of diospyrin have also been prepared and tested with a view to reducing its pronounced cytotoxic effects towards the animal host. (HAZRA, Banasri et al: 'New diospyrin derivatives with improved tumour inhibitory activity towards *Ehrlich ascites carcinoma*', Med. Sci. Res (1994), 22(5), 351-3, XP000978374).

The earlier isolation of phthiocol (3-hydroxy-2-methyl-1,4-naphthoquinone) from the acetone-soluble fat fraction of tubercle bacilli and the confimiation that it had antituberculous activity against H-37 R.V. strain *in vitro* and in mice has also prompted the synthesis of some 2-alkyl-3-hydroxy-1.4-naphthoquinone-4-aryl(aroyl)hydrazones as possible tuberculostatic agents. (ROUSHDI, I.M. et al: 'Synthesis of 1.4-naphthoquinones-4-aryl(aroyl)hydrazones of potential antimicrobial activity', Pharmazie, (1976) 31(12) 856-9, XP000971908).

2- and 3-substituted α-naphthoquinones have also been synthesised and a number of these have been shown to have antibacteriostatic activity against *Mycobacterium tuberculosis*. (OERIU, I: 'Relation between the chemical structure and the antitubercular effect of alpha-naphthoquinone derivatives substituted in 2 and 3 positions', Pharmazie, (1961 May) 16 266-72., XP000971910; and OERIU I: 'Zusammenhänge zwischen der chemischen Stuktur und der antituberkulösen Wirklung der In Stellung 2 und 3 substituieren Derivate des alpha-Naphthochinons', Pharmazie, no. 16, 1961, pages 320-327, XP002078405 ISSN: 0031-7144).

### DESCRIPTION OF PREFERRED EMBODIMENTS

The present invention is directed at the use of naphthoquinone derivatives of Formula 1a and 1b 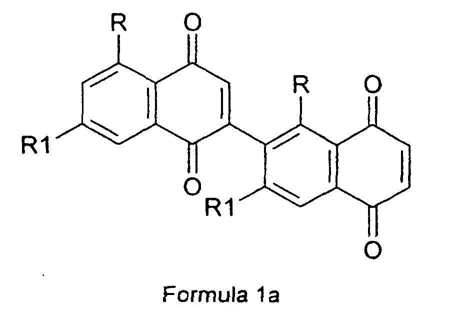 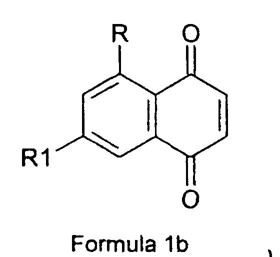 wherein R represents an OH group and R₁ represents a methyl group or pharmaceutically acceptable salts thereof, in the manufacture of a medicament for use in the treatment and/or control of tuberculosis caused by *Mycobacterium tuberculosis*.

Diospyrin and methyljuglone, naphthoquinone derivatives of Formula 1a and Formula 1b, respectively, in which R is OH and R1 is a methyl group, have been found to inhibit several antibiotic resistant as well as antibiotic susceptible strains of *Mycobacterium tuberculosis.*

An extensive research program was undertaken in order to identify antituberculosis agents that can readily and simply be produced from local resources.

Twenty South African medicinal plants used to treat pulmonary diseases were screened for activity against drug-resistant and sensitive strains of *M*. *tuberculosis*. A preliminary screening of acetone and water plant extracts, against a drug-sensitive strain of *M. tuberculosis;* H37Rv, was carried out by the agar plate method. Fourteen of the 20 acetone extracts showed inhibitory activity at a concentration of 0.5 mg/ml against this strain. Acetone as well as water extracts of *Cryptocarya latifolia*, *Euclea natalensis, Helichrysum melanacme*, *Nidorella anomala* and *Thymus vulgaris* inhibited the growth of *M. tuberculosis*. Given the activity of 14 acetone extracts at 0.5 mg/ml against the drug-sensitive strain by the agar plate method a further study was carried out employing the rapid radiometric method to confirm the inhibitory activity. These active acetone extracts were screened against the H37Rv strain as well as a strain resistant to the drugs, isoniazid and rifampicin. The minimal inhibitory concentration of *Croton pseudopulchellus*, *Ekebergia capensis*, *Euclea natalensis*, *Nidorella anomala* and *Polygala myrtifolia* was 0.1 mg/ml against the H37Rv strain by the radiometric method. Extracts of *Chenopodium ambrosioides*, *Ekebergia capensis*, *Euclea natalensis*, *Helichrysum melanacme*, *Nidorella anomala* and *Polygala myrtifolia* were active against the resistant strain at 0.1 mg/ml. Eight plants showed activity against both the strains at a concentration of 1.0 mg/ml.

The following procedure was developed by the applicant for the isolation of diospyrin and methyljuglone from E. *natalensis* and other species in this genus, as well as any other plants that may synthesise diospyrin or methyljuglone or other quinone derivatives.

### 1. Identification of plant species

Roots and the aerial plant parts of *E*. *natalensis* were collected near Durban and identified at the HGWJ Schweickerdt Herbarium of the University of Pretoria and also at the herbarium of the National Botanical Institute, Pretoria.

### 2. Extraction

Dried roots of *E natalensis* were ground to a powdery form with a dry mill and extracted over 48 hours with acetone. The extract was filtered and concentrated to dryness at reduced pressure on a rotary evaporator.

### 3. Thin layer chromatography

A direct antibacterial bioassay (Dilika & Meyer 1996) on TLC-plates was employed to speedup the activity guided isolation of the antituberculosis compounds. *M. tuberculosis* cannot be tested in this way because of its very slow growth rate. The direct antibacterial bioassays of the acetone extract were done on TLC plates (Merck) developed with chloroform-hexane (1:1). After development, the TLC plates were dried and sprayed with a 24 hr old *Staphylococcus aureus* culture in nutrient broth. After 24 hr incubation, the plates were sprayed with an aqueous solution of 2mg/ml p-iodonitrotetrazolium violet to visualise the bacterial cells. The plates were then reincubated at 37°C for 2-3 hours.

Two zones of bacterial growth inhibition could be seen on TLC plates sprayed with *S. aureus.* Activity was more pronounced in the R_{f} 0.30 zone (chloroform-hexane (1:1)) than in the R_{f} 0.54 zone.

### 4. Column chromatography

The crude extract of the plant was dried, its mass determined and resuspended in chloroform. Column chromatography was performed on silica gel 60 using chloroform as eluent. The antibacterial fractions collected were then subjected to a Sephadex LH-20 column chromatography using ethanol as eluent. The fractions collected were again tested for antibacterial activity on TLC to detect the fractions containing the active compounds of R_{f} 0.30 and R_{f} 0.54.

### 5. High performance liquid chromatography

The compounds were further purified by HPLC utilising an analytical Phenomenex reverse phase 250x4.60 mm column, at a flow rate of 1.0 ml/min, oven temp. 40°C and a wavelength of 206nm. An ethanol-water (50:50) solution was employed as mobile phase. The pure compounds were once again subjected to a Sephadex LH-20 column chromatography and proved to be pure. The chemical structures were confirmed by ¹H and ¹³C nmr and ms to be: 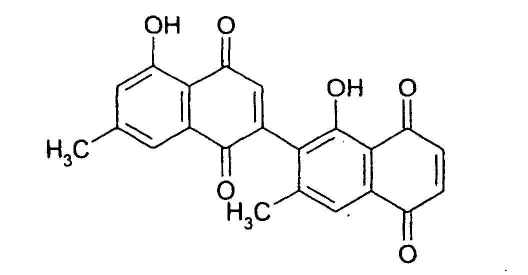 Diospyrin (5,5' dihydroxy 7,7' binaphthoquinone); C₂₂H₁₄O₆. Molecular weight: 374.35 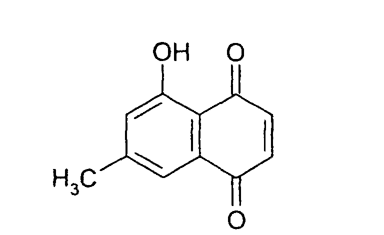 7-methyljuglone (5-hydroxy-7-methyl-1,4-naphtoquinone); C₁₁H₈O₃ Molecular weight: 188.19

The effect of diospyrin and methyljuglone on the growth of the sensitive strain (H37Rv) and resistant strains of *Mycobacterium tuberculosis* as determined by the radiometric method are set out in Table 1 and Table 2.

**TABLE 1**

| Effect of diospyrin on the growth of the sensitive strain (H37Rv) and resistant strains of *Mycobacterium tuberculosis* as determined by the radiometric method. | | | |
|---|---|---|---|
| *Mycobacterium tuberculosis* strains | MIC (mg/ml) | ΔGI^{a} values of plant extracts (mg/ml) | ΔGI values of the control vial (mg/ml) |
| H37 sensitive strain | 0.1 | -1 ± 1.41 | 20 ± 4.24 |
| 2 drug resistant strain (res. to Isoniazid and rifampicin). | 0.1 | 3.5 ± 0.70 | 25 ± 7.07 |
| 3 drug resistant strain (res. to streptomycin, isoniazid and ethambutol), | 0.1 | 4 ± 2.12 | 29 ± 1.41 |
| 4 drug resistant strain (res. to streptomycin, isoniazid, rifampicin and ethambutol). | 0.1 | 5 ± 2.82 | 25 ± 2.82 |
| 5 drug resistant strain.(res to isoniazid, streptomycin, rifampicin, thiacetazone and cyclocerine). | 0.1 | 10 ± 1.41 | 22.5 ± 3.53 |
| 6 drug resistant strain (res. to isoniazid, rifampicin, ethionamide, terizidone, thiacetazone and ofloxacin). | 0.1 | 9 ± 2.82 | 30 ± 1.0 |
| 7 drug resistant strain.(res to isoniazid, streptomycin, ethambutol, kanamycin, rifampicin, and ethionamide) | 0.1 | 13.5 ±3.2 | 28 ± 3.1 |

| | | | |
|---|---|---|---|
| ^{a}ΔGI values are means ± s.d. | | | |

**TABLE 2**

| Effect of 7-methyljuglone as a single agent and in combination with diospyrin on the growth of the sensitive strain (H37Rv) and resistant strains of *Mycobacterium tuberculosis* as determined by the radiometric method. | | | | | |
|---|---|---|---|---|---|
| *Mycobacterium tuberculosis* strains | Lab reference no. | Compound(s) | MIC^{a}(µg/ml) | ΔGI^{b}values of plant extracts | ΔGI values of the control vial |
| H37Rv sensitive strain | ATCC27294 | 7-methyljuglone | 50 | 0 ± 1 | 15 ± 3.78 |
| Two drug (isoniazid and rifampicin) resistant strain | CCK028469V | 7-methyljuglone | 50 | 0 ± 0 | 30 ± 4.94 |
| H37Rv sensitive strain | ATCC27294 | Diospyrin + 7-methyljuglone | 10 | 3 ± 1 | 15 ± 3.78 |
| Two drug (Isoniazid and rifampicin resistant strain) | CCK028469V | Diospyrin + 7-methyljuglone | 10 | 3.33 ± 3.05 | 30 ± 4.94 |

| | | | | | |
|---|---|---|---|---|---|
| ^{a}Minimal inhibitory concentration | | | | | |
| ^{b}ΔGI values are means ± s.d. | | | | | |

The results show that diospyrin and methyljuglone control the *Mycobacterium tuberculosis* bacterium effectively. Oral administration of diospyrin or methyljuglone in an appropriate pharmaceutical composition with suitable diluents and carriers will typically be used to treat or control tuberculosis. This will be by way of tablet, liquid or similar oral dosage form, as diospyrin and methyljuglone are readily absorbed intestinally.

However, it is believed that diospyrin or methyljuglone administered intravenously or intramuscularly will also be absorbed effectively through blood vessels and the blood stream of a patient. Transdermal administration, via a plaster or similar transdermal administration vehicle, is also a possibility.

A combination treatment of diospyrin and methyljuglone, which may be more effective than singular treatments of the two naphthoquinones, is also envisaged.

The applicant believes that it may be possible to increase the concentration of diospyrin, methyljuglone and other quinones in *E. natalensis* or similar species by phytoalexic stimulation or by the biotechnological manipulation of tissue cultures and/or intact plants.

Quinones are generally synthesised from catechol (1,2-quinones) or hydroquinone (1,4-quinones) by mild oxidation.

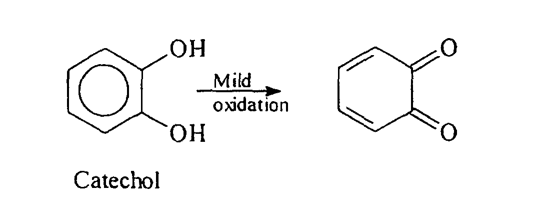

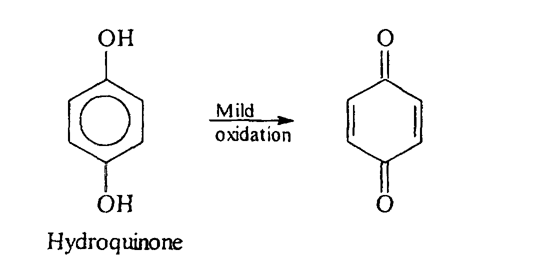

As far as the applicant has been able to establish, diospyrin has been synthesised once in a laboratory (Yoshida, M and Mori, K. 2000. European Journal of Organic Chemistry pages 1313 - 1317). However, similar binapthoquinones can also be synthesised by the reaction of plumbagin (94mg in methanol, 10ml) and its hydroquinone (190mg in methanol, 14ml), buffered in phosphate to pH 6.8 at 30°C. (Sankaram et al. 1975; Kumari et al. 1982).

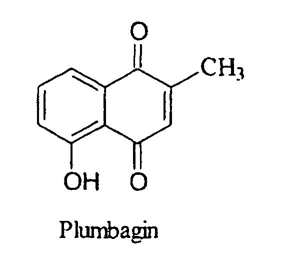

It is believed that diospyrin, methyljuglone and related naphthoquinone derivatives are viable alternatives to conventional drugs in the treatment and control of tuberculosis in humans.

## Claims

1. The use of a naphthoquinone derivative having the Formula 1a or Formula 1b: 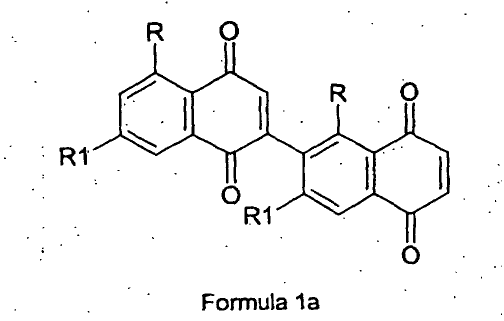 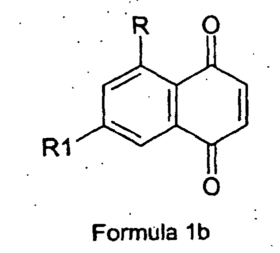 wherein R represents an OR group and Rl represents a methyl group, or pharmaceutically acceptable salts thereof, in the manufacture of a medicament for use in a method of treating and/or controlling tuberculosis in a patient caused by mycobacterium tuberculosis.

2. The use according to claim 1 wherein the naphthoquinone derivative is 5,5' dihydroxy 7,7'binaphthoquinone (diospyrin) or 5-hydroxy-7-methyl-1,4-naphtoquinone (methyljuglone), or a mixture thereof.

## Patentansprüche

1. Verwendung eines Naphthochinon-Derivats der Formel 1 a oder Formel 1 b: 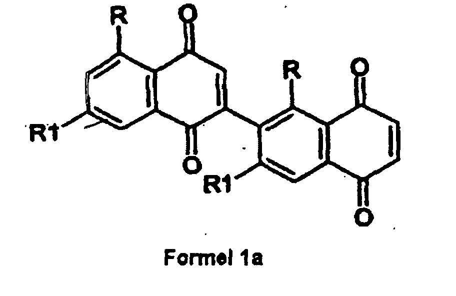 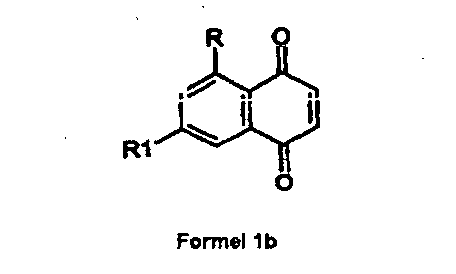 worin R eine OH-Gruppe und R1 eine Methylgruppe ist, oder von pharmazeutisch annehmbaren Salzen davon, bei der Herstellung eines Arzneimittels zur Verwendung in einem Verfahren zur Behandlung und/oder Kontrolle von Tuberkulose in einem Patienten, hervorgerufen durch Mycobacterium tuberculosis.

2. Verwendung nach Anspruch 1, worin das Naphthochinon-Derivat ein 5,5'-Dihydroxy-7,7'-binaphthochinon (Diospyrin) oder 5-Hydroxy-7-methyl-1,4-naphthochinon (Methyljuglon) oder einer Mischung davon ist.

## Revendications

1. Utilisation d'un dérivé de naphtoquinone présentant la formule la ou la formule 1b : 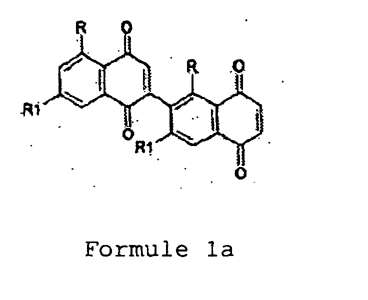 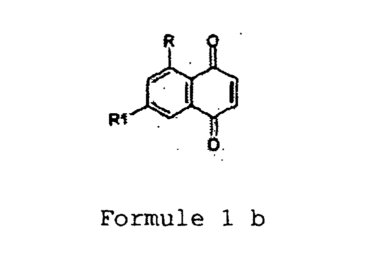 dans lesquelles R représente un groupe OH et R1 représente un groupe méthyle, ou un sel pharmaceutiquement acceptable de celui-ci, dans la fabrication d'un médicament à utiliser dans un procédé de traitement et/ou de contrôle de la tuberculose produite par mycobacterium tuberculosis chez un patient.

2. Utilisation selon la revendication 1, dans laquelle le dérivé de naphtoquinone est le 5,5' - dihydroxy-7,7'-binaphtoquinone (diospyrine) ou le 5-hydroxy-7-méthyl-1,4-naphtoquinone (méthyljuglone) ou un mélange de ceux-ci.
